# EUROPEAN PATENT APPLICATION

(11) **EP 1 588 988 A1**
(43) Date of publication of application: **26.10.2005**
(21) Application number: 04076061.3
(22) Date of filing: 02.04.2004
(51) Int. Cl.: C02F 3/28, B65D 88/12

(54) **Three-phase separator for an upflow sludge bed reactor**

(71) Applicant: BIOTHANE SYSTEMS INTERNATIONAL B.V., NL-2623 EW Delft (NL)
(72) Inventor: Las Vos, Hendrik Richard Paul, 2982 CK Ridderkerk (NL); Otten, Michael Johannes, Pondok Indah Jakarta 12310 (IN)
(74) Representative: Winckels, Johannes Hubertus F.

(57) **Abstract**

The invention is directed to a three phase separator for an upflow anaerobic sludge bed reactor, said separator at least consisting of a structure with the dimensions of an ISO-container, wherein the bottom and the top of the said container or structure is open and separating means for gas-liquid-solid separation are present inside and/or on top of the said container or structure.

## Description

The present invention is in the area of anaerobic waste water purification and more in particular anaerobic sludge bed systems, such as Upflow Anaerobic Sludge Bed (CTASB) systems and more in particular in the construction of a three-phase separator for an upflow anaerobic sludge bed reactor.

Anaerobic sludgebed reactor systems utilise consortia of anaerobic bacteria to convert pollutants in wastewater to biogas. These anaerobic bacteria grow in aggregates, often referred to as granular biomass. The systems, known as UASB, EGBS, IC etc. are characterised by low net biomass production (typically 2-3 % of converted COD) as a result of the low net yield of anaerobic bacteria involved. This is a significant system advantage, as the excess biomass developed in wastewater treatment systems has to be disposed as a solid waste, at significant cost.

The purification process generally comprises a system, wherein fresh wastewater is introduced in the bottom of an upflow reactor, containing dispersed biomass in (partly purified) wastewater. During the anaerobic purification process, biogas is produced and a mixture of water, biomass and gas flows upward in the reactor. Before purified wastewater can be discharged, a gas-liquid-solid separation has to take place. This is generally done using three phase separators, which are placed, at the top of the reactor. Various constructions are known, most of them are based on 'hood-like' constructions that are overlapping. Any biogas that is produced in the reactor under the three phase separator is collected under these hoods and a gas free, relatively quiet settling zone is created above the hoods. As a result sludge will settle and is transported back to the reactor section under the three-phase separator. The liquid fraction becomes largely sludge free and can be discharged via collection gutters at the top of the separator (the effluent).

Typical examples of three phase separators can be found in the US patent Nos 4 165 285 and 4 253 956, as well as in EP-A . 0 244 029. Further information may also be found in Feasibility of the Upflow Anaerobic Sludge Blanket (UASB) process, Dr. Ir. G lettinga et al. Proceedings 1979 NATIONAL CONFERENCE ON ENVIRONMENTAL ENGINEERING, ASCE/San Francisco, Ca/ July 9-11, 1979 and UASB process design for various types of wastewater. Lettinga et al. 1991, Water Science & Technology 24(8), 87-107. The contents of these documents is incoprporated herein by way of reference.

The construction of a separator usually takes place at a location different from the site of the wastewater purification plant to be build. Due to the size and weight of the unit, transport and installation costs play an important role in the total investments for such a plant. Especially when the plant is to be construed in a remote location or in a country that has limited construction possibilities, the transport and the costs thereof play an important role.

It is an object of the invention to provide a three-phase separator unit for an upflow anaerobic sludge bed waste water purification system, which unit is constructed in such a way, that it is easy to transport, and also easy to install at the location of the plant. As a consequence, the total investment costs of the installation are reduced.

The present invention is based thereon, that the external structure of the three-phase separator and especially the foot,side and top print of the separator is based on a standard ISO container or on the dimensions of a standard ISO container. The gas-liquid-solid separating means are situated inside and/or on top of the structure

The invention is accordingly characterized by a three-phase separator for an upflow anaerobic sludge bed reactor, said separator at least consisting of a structure in accordance with the dimensions of a standard ISO-container, wherein the bottom and the top of the said container or structure are open and separating means for gas-liquid-solid separation are present inside and/or on top of the said structure.

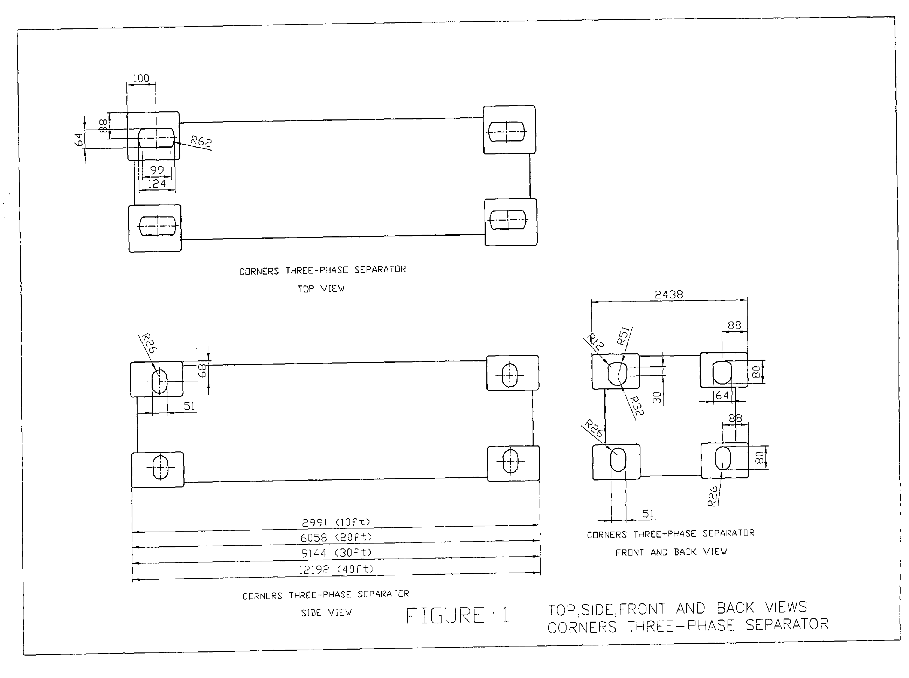

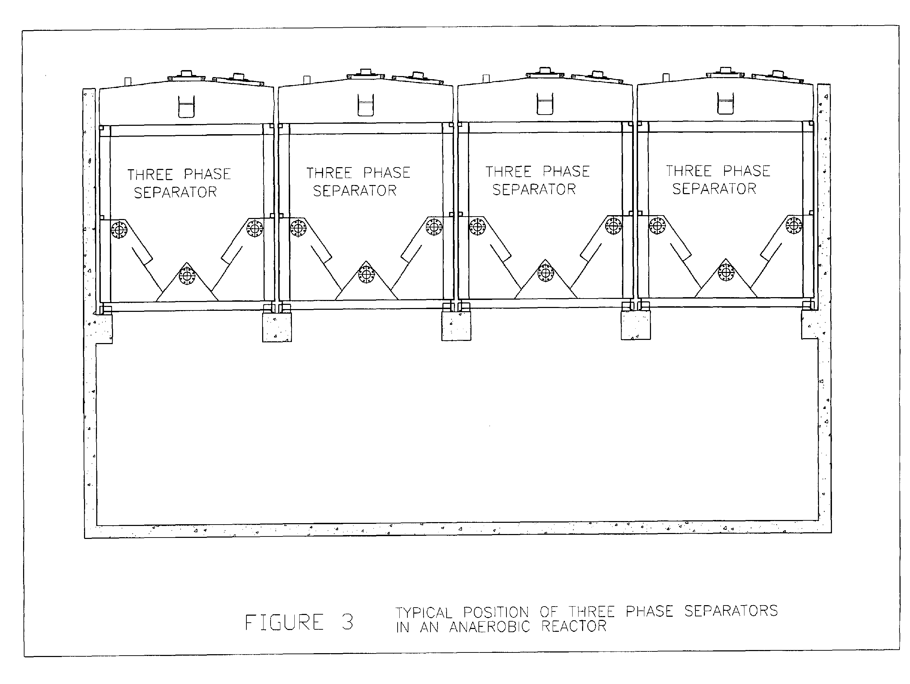

Depending on the required size of the separator it is possible to use 10, 20, 30 or 40 ft ISO-containers or structures in accordance with the dimensions of this type of ISO-containers (see figure 1). It is possible to use two or more separators next to each other, as shown in Figure 3.The use of these standard containers, or a construction based on the standard ISO containers sizes (ISO 668 (4^{th} edition- 1988), has the distinct advantage that the basic structure of the separator can easily and cheaply be shipped as container, using container ships or trucks to transport it to the required location. In a further embodiment, all the internals for the separator are construed in such a manner, that they fit inside the container or the structure with dimensions of a standard container, thereby enabling the transport of the total of the separator as a standard container across the road/water. The important sizes (in mm) have been shown in Figure 1. It is to be noted that the height of the container is less important, as various heights are possible. This height will generally be between 2000 and 3000 mm.

The internals of the separator form the actual gas-liquid-solid separating means, as well as the means for withdrawal of purified waste water (the effluent), the collection of the produced biogas, as well as the recycling of the settled solids and part of the water to the reactor section underneath the separator.

The invention is also directed to a waste water purification plant of the upflow sludge reactor type, comprising a separator as described herein. The invention is also directed to a process for constructing a waste water purification plant including at least one three-phase separator as defined herein and an upflow sludge bed reactor, said process comprising constructing the said three phase spearator at a location removed from the location of the waste water purification plant, transporting the said three-phase separator to the location of the waste water purification plant and placing and constructing the three-phase separator on top of the preconstructed upflow sludge bed reactor.

The invention is now elucidated on the basis of a number of figures, showing the standard ISO container top, side and corners front/back views and the dimensions to be used for three phase separators (figure 1).

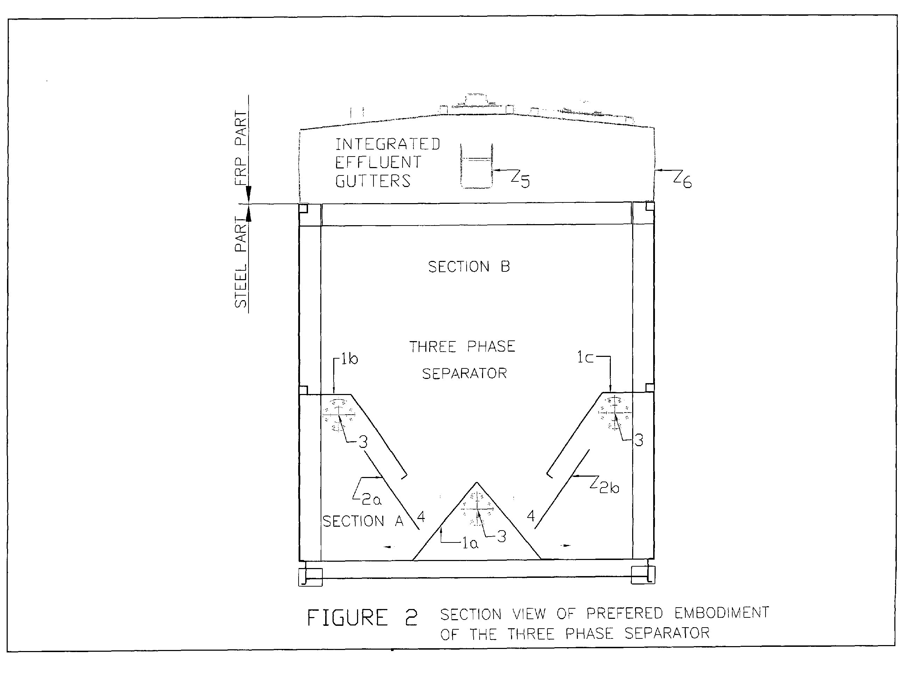

The preferred embodiment of the three phase separator (figure 2).

A typical positioning of the three phase separator(s) in an anaerobic reactor (figure 3).

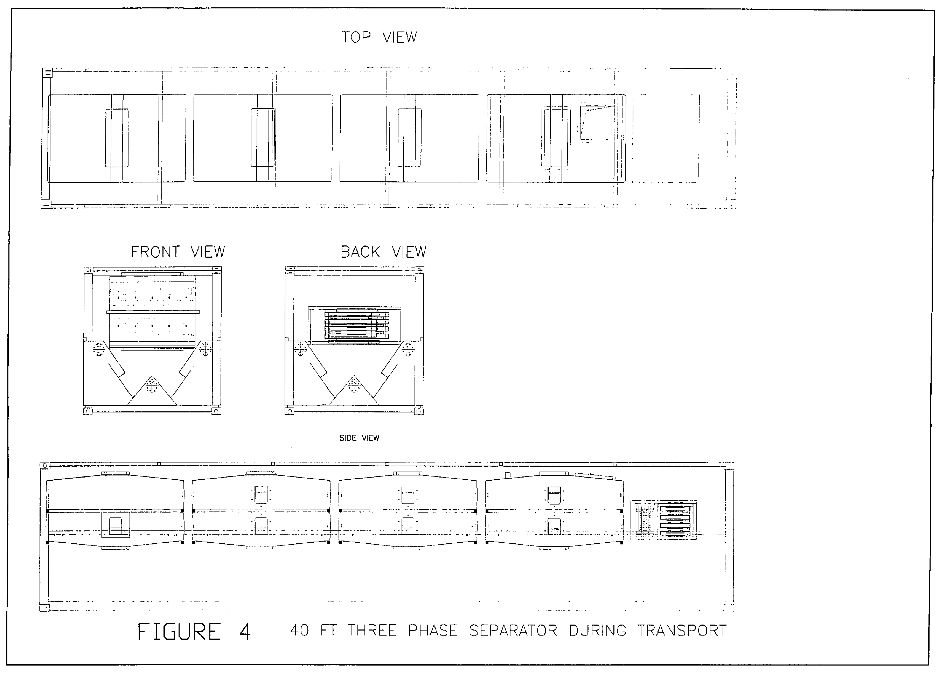

The preferred embodiment of the three-phase separator during transport (figure 4).

In a preferred embodiment, the separating means are as in figure 2.

The three-phase separator consists of two main sections.

First of all the biogas is separated from the water and sludge in section A.

To accomplish that the bottom part of the three phase separator consists of overlapping biogas collection hoods (1a,b and c) and baffle plates (2a and 2 b) One hood in the center (1a) and two hoods at the sides (1b and 1c). Biogas will be collected under these hoods and piped away via openings 3 in the endplate of the three-phase separator.

The second section of the three-phase separator is the actual settler in which the sludge is separated from the water (section B).

To enable settled sludge to return to the reactor section openings 4 are present in between the hoods and the baffle plates.

In the top of the three-phase separator means 5 are present (gutters) to collect and discharge the water (the effluent).

In a preferred embodiment these means 5 are integrated with the covers 6 of the three-phase separator, which are positioned on top of the container or structure.

During transport the covers with the integrated effluent gutters are stored inside the container or structure body (see figure 4).

The materials to be used for these internals, can be the standard materials, such as steel, coated steel, plastics such as polypropylene, fibre reinforced plastics (epoxy and/or unsaturated polyester resins) and the like. More in particular, the steel materials are used for underwater applications, whereas the plastics materials are used above the water level. The advantage thereof is that the strength of steel is used to give a rigid structure to the three-phase separator, whereas for parts above water, which are sensitive for corrosion, non corrosive materials like plastics can be used.

The separator of the present invention is placed in the top of an upflow anaerobic sludge bed reactor, for example as described in figure 3. The cross-section of the reactor will be the same or bigger as the cross-section of the separator (s).The cross-section will usually be rectangular but can also be square, or round.

The separator will have a water-tight connection with the reactor, as the purified wastewater (effluent) will generally be withdrawn from the separator.

## Claims

1. Three-phase separator for an upflow sludge bed reactor, said separator at least consisting of a structure with the dimensions of a an ISO-container, wherein the bottom and the top of the said structure is open and separating means for gas-liquid-solid separation are present inside and/or on top of the said container or structure .

2. Three-phase separator according to claim 1, wherein the said container or structure is e in accordance with a 10 ft, a 20 ft, a 30 ft or a 40 ft container according to ISO 668.

3. Upflow anaerobic sludge bed reactor wherein two or more three-phase separators according to claim 1 or 2, are used, which are positioned against each other at the side walls.

4. Three-phase separator according to claim 1-3, wherein gas hood type of gas-liquid separating means are provided in the lower half of the container or structure which are extending in the length of the container or structure.

5. Three-phase separator according to claim 1-4, wherein effluent collecting means consisting of a water collection gutter are provided on top of the container or structure and are combined with the covers for the separator in one structure.

6. Three-phase separator according to claim 1-5, wherein the said separating means for gas-liquid-solid separation are constructed from steel, coated steel, plastics material, such as glass fibre reinforced plastic material or polypropylene.

7. Three-phase separator according to claim 1-6, wherein the underwater parts of the said separating means are constructed from steel and/or coated steel.

8. Three-phase separator according to claim 1-7, wherein the parts of the said separating means which are not underwater are constructed from plastics material.

9. Three-phase separator according to claim 1-8, wherein the size and construction of the said effluent collection means and separator covers are such, that these can be stored inside the container or structure during transport.

10. Waste water purification plant, more in particular of the upflow anaerobic sludge bed type, comprising an upflow reactor and separating means in the top of said reactor, the separating means comprising the separator of any one of the claims 1-9.

11. Process for constructing a waste water purification plant including at least one three-phase separator as defined in claim 1-9 and an upflow sludge bed reactor, said process comprising constructing the said three phase spearator at a location removed from the location of the waste water purification plant, transporting the said three-phase separator to the location of the waste water purification plant and placing and constructing the three - phase separator on top of the preconstructed upflow sludge bed reactor.
